## Europäisches Patentamt
## European Patent Office
## Office européen des brevets

(19)

(11) Veröffentlichungsnummer: **0 137 269**
**B1**

(12) # EUROPÄISCHE PATENTSCHRIFT

(45) Veröffentlichungstag der Patentschrift:
18.03.87

(51) Int. Cl.⁴: **C 12 Q 1/56**

(21) Anmeldenummer: **84110145.4**

(22) Anmeldetag: **24.08.84**

(54) Verfahren zur gleichzeitigen Bestimmung von Fibrinogen und Fibrinogen-Spaltprodukten im Plasma.

(30) Priorität: **25.08.83 DE 3330699**

(43) Veröffentlichungstag der Anmeldung:
**17.04.85 Patentblatt 85/16**

(45) Bekanntmachung des Hinweises auf die Patenterteilung:
**18.03.87 Patentblatt 87/12**

(84) Benannte Vertragsstaaten:
**AT BE CH DE FR GB IT LI LU NL SE**

(56) Entgegenhaltungen:
**AT-B-353 423**
**DD-A-158 954**
**US-A-4 245 039**

(73) Patentinhaber: **BOEHRINGER MANNHEIM GMBH,
Patentabteilung, Abt. E Sandhofer Strasse 112-
132 Postfach 31 01 20, D-6800 Mannheim 31
Waldhof (DE)**

(72) Erfinder: **Becker, Udo, Dr.rer.nat., Birkenweg 2,
D-3550 Marburg (DE)**
Erfinder: **Roeschlau, Peter, Dr.rer.nat.,
Schönegertstrasse 4, D-8124 Seeshaupt (DE)**

(74) Vertreter: **Weickmann, Heinrich, Dipl.- Ing.,
Patentanwälte Dipl.- Ing. H.Weickmann Dipl.-
Phys.Dr. K.Fincke Dipl.- Ing. F.A.Weickmann
Dipl.- Chem. B. Huber Dr.- Ing. H. Liska Dipl.-
Phys.Dr. J. Prechtel Möhlstrasse 22, D-8000
München 80 (DE)**

EP 0 137 269 B1

LIBER, STOCKHOLM 1987

**Beschreibung**

Die Bestimmung von Fibrinogen und Fibrinogen-Spaltprodukten (FSP) im Plasma ist wichtig im Rahmen der Untersuchung von Gerinnungsstörungen. Eine Methode, die die gleichzeitige Bestimmung beider Parameter in Plasma gestatten würde, ist bisher nicht bekannt. Die beiden Parameter müssen jeweils unabhängig bestimmt werden, da sich die Effekte überlagern.

Zur Bestimmung des Fibrinogens sind sowohl immunologische Methoden als auch Gerinnungstests bekannt. Die immunologischen Methoden sind unspezifisch und vermögen nicht zwischen Fibrinogen und FSP zu differenzieren. Sie weisen darüber hinaus diagnostisch schwerwiegende Nachteile auf und haben daher in der Praxis keine Bedeutung erlangt.

Bei den Gerinnungstests wird der Fibrinogengehalt durch Zeitmessung der Gerinnselbildung ermittelt. Die größte Bedeutung hat die in Acta haemat. 17, 237-246 (1957) beschriebene Methode nach Clauss erlangt. Bei diesem Verfahren wird einem verdünnten Plasma, also einer schwachen Fibrinogenlösung, konzentrierte Thrombinlösung zugesetzt. Die Thrombinmenge beträgt hierbei etwa 500 U/ml Plasma. Mit Hilfe einer Kalibrierungskurve wird die Zeit bis zum Auftreten eines Gerinnsels in Beziehung zum Fibrinogengehalt der Probe gesetzt.

Weiter sind Gerinnungstests bekannt, bei denen die Trübungsbildung im Verlauf der Gerinnung photometrisch registriert wird und zwar entweder als Endpunktmethode oder als kinetische Methode.

Schließlich sind auch Methoden bekannt, bei denen das gebildete Gerinnsel isoliert und sein Eiweißgehalt chemisch bestimmt wird. Hierbei wird die Probe mit Thrombin umgesetzt, das gebildete Gerinnsel isoliert, gewaschen, getrocknet und dann der Eiweißgehalt oder das Gewicht ermittelt.

Methoden zur Bestimmung von FSP beruhen auf dessen gerinnungshemmender Wirkung.

Bekannt ist die immunologische Bestimmung mit Antikörpern gegen Fibrinogen oder FSP. Da jedoch keine Antikörper bekannt sind, die nur mit FSP, aber nicht mit Fibrinogen reagieren und umgekehrt auch Antikörper gegen Fibrinogen stets auch mit FSP reagieren, erfordert diese Bestimmung vorher eine Trennung von Fibrinogen und FSP.

Daneben wird auch die gerinnungshemmende Wirkung von FSP direkt zur Bestimmung ausgenützt. Hierbei wird die Probe mit Thrombin umgesetzt und die Zeit bis zum Eintritt der Gerinnung gemessen, da FSP eine thrombinhemmende Wirksamkeit ausübt.

Aus obigem geht hervor, daß zwar im Prinzip die gleiche Technik sowohl für die Fibrinogen- als auch für die FSP-Bestimmung angewendet wird, nämlich Umsetzung von Plasma mit Thrombin und Bestimmung der Zeit bis zum Eintritt der Gerinnung. Jedoch besteht ein grundsätzlicher Unterschied in der Wahl der Enzymkonzentration in Bezug auf die eingesetzte Probe. Clauss hatte als erster erkannt, daß die Gerinnungszeit einer mit Thrombin umgesetzten Plasmaprobe dann dem Fibrinogengehalt proportional ist, wenn ein genügend großes Verhältnis von Thrombin zur Probemenge gewählt wird, im speziellen Fall 500 E/ml Plasma. Hauptsächliche Störfaktoren sind hierbei Antithrombine, also solche Substanzen, welche die Thrombinwirkung hemmen. Bekannt sind Antithrombin III, Antithrombin BM und $\alpha_2$-Makroglobulin sowie FSP.

Bei allen bekannten Fibrinogen-Bestimmungsmethoden wird daher versucht, den Einfluß der genannten Störfaktoren gering zu halten, zumeist durch entsprechende Erhöhung der Enzymmenge. Auch ist es bekannt, den Einfluß der Störfaktoren, der in Gegenwart von geringen Mengen Heparin verstärkt wird, durch Heparin-Antagonisten zu verringern.

Bestimmt man hingegen FSP, so wird die Thrombinkonzentration sehr niedrig gehalten, da andernfalls die Fibrinogenkonzentration das Resultat stark beeinflußt. Unter diesen Umständen aber nimmt der Einfluß der plasmatischen Inhibitoren zu, so daß diese Methode nicht besonders spezifisch ist.

Aus J. Lab. Clin. Meth. 58, 477 (1961) ist auch eine turbidimetrische Fibrinogenbestimmung bekannt. Hierbei handelt es sich um ein Endpunktverfahren unter Einsatz eines Standards, welches infolge der langen Inkubationszeit von 20 Minuten eine Automatisierung nicht zuläßt. Das in Clin. Chem. 29, 614 (1983) beschriebene Verfahren beseitigt diesen Nachteil durch kinetische Messung mit einem Zeitintervall von etwa 17 sec. Hier wird jedoch eine Mindestaktivität von 12 NIH U Thrombin/ml Plasma benötigt und man erhält eine nichtlineare Eichbeziehung zwischen Meßsignal und Fibrinogenmenge, die nur aufwendig ausgewertet werden kann. Dieses Verfahren ist daher für einen Routinetest nicht geeignet.

Der Erfindung liegt daher die Aufgabe zugrunde ein Verfahren zu schaffen, welches die oben geschilderten Nachteile der bekannten Verfahren vermeidet, insbesondere kurze Meßzeiten mit geringen Enzymaktivitäten benötigt und sowohl Fibrinogen als auch FSP gleichzeitig zu bestimmen gestattet.

Diese Aufgabe wird erfindungsgemäß gelöst durch ein Verfahren zur gleichzeitigen Bestimmung von Fibrinogen und Fibrinogen-Spaltprodukten im Plasma, welches dadurch gekennzeichnet ist, daß man 0,01 bis 1 U eines Schlangengiftenzyms mit thrombinartiger Wirksamkeit je ml Probeplasma zusetzt, dann die Zeitdauer zwischen dem Enzymzusatz und dem Beginn einer Trübungsbildung als Maß für die Menge an Fibrinogenspaltprodukten bestimmt und anschließend die Geschwindigkeit der Trübungsbildung als Maß für die Fibrinogenmenge mißt.

Die Erfindung beruht auf der Erkenntnis, daß bei Ersatz des Thrombins durch ein Schlangengiftenzym in der angegebenen niedrigen Aktivität die bekannten Störfaktoren praktisch ausgeschaltet werden und sowohl die Zeitdauer zwischen Schlangengiftenzymzusatz und Beginn der Trübungsbildung als auch die Geschwindigkeit der Trübungsbildung selbst jeweils direkt proportional der Menge an FSP bzw. Fibrinogen sind. Die besten Ergebnisse werden bei der Erfindung mit einer Enzymkonzentration zwischen 0,05 und 0,5 U erzielt.

Geeignete Schlangengiftenzyme sind solche mit einer thrombinartigen Wirksamkeit. Bevorzugt verwendet man Batroxobin, das Giftenzym der malayischen Grubenotter (agkistrodon rhodostoma) oder das Giftenzym der Kupferkopfschlange (agkistrodon contortrix).

In einer bevorzugten Ausführungsform des erfindungsgemäßen Verfahrens arbeitet man in Gegenwart von 0,5 bis 4 Gew.-% eines wasserlöslichen Polyalkohols. Der Polyalkohol erhöht die Gleichmäßigkeit der Trübungsbildung. Beispiele für geeignete Polyalkohole sind Stärke, Dextran, Polyvinylalkohol, Polyethylenglykol, Zucker oder Zuckerpolymerisate. Besonders günstige Ergebnisse wurden mit einem Polyethylenglykol eines Molekulargewichts zwischen 4000 und 22000 erhalten.

Das erfindungsgemäße Verfahren wird bei pH-Werten zwischen 7,0 und 9,0, vorzugsweise bei pH 7,3 bis 7,8 durchgeführt. Wesentlich ist jedoch allein, daß der pH-Wert im physiologischen Bereich liegt. Die Pufferkonzentration liegt zweckmäßig zwischen 0,05 und 0,3 Mol/l Pufferlösung. Alle im angegebenen Bereich puffernden und physiologisch verträglichen Puffersubstanzen sind im Rahmen der Erfindung geeignet. Beispielsweise seien genannt Tris-/HCl und Phosphat-Puffer.

In an sich bekannter Weise wird außerdem in Gegenwart eines löslichen Kalziumsalzes gearbeitet, welches Ca++-Ionen in einer Menge von 1 bis 20 mMol/l liefert.

Schließlich erwies sich auch ein Zusatz von Detergenzien wie z. B. Brij 35 als zweckmäßig. Geeignete Mengen liegen zwischen 0,1 und 2 %. Beispiele für geeignete Kalziumsalze sind Kalziumhalogenide wie Kalziumchlorid, Kalziumacetat und dergleichen.

Ein weiterer Gegenstand der Erfindung ist ein Reagenz zur Durchführung des oben beschriebenen Verfahrens, welches dadurch gekennzeichnet ist, daß es 0,01 bis 1 U Schlangengiftenzym mit thrombinartiger Wirksamkeit je ml Probeplasma, 0,5 bis 4 Gew.-% eines wasserlöslichen Polyalkohols, 1 bis 20 mMol/l Ca++ und 0,05 bis 0,3 Mol/l Puffer pH 7,0 bis 9,0, jeweils bezogen auf gebrauchsfertige Lösung, sowie gegebenenfalls ein Detergenz enthält. Das erfindungsgemäße Reagenz kann als Mischung der trockenen Substanzen oder auch in Form einer konzentrierten oder gebrauchsfertig verdünnten Lösung vorliegen.

Durch die Erfindung wird erreicht, daß in einer Analyse mit sehr geringem Zeitbedarf und einer einzigen Kalibrierung sowohl Fibrinogen als auch FSP gleichzeitig bestimmt werden können, wobei Störungen durch Antithrombine ausgeschaltet sind. Gleichzeitig werden nur minimale Enzymmengen benötigt.

In AT-B-353423 wird ein Verfahren zur Bestimmung von Fibrinogen, unter Verwendung von Schangengift als Fibrinogenase, beschrieben. Als Maß für den Fibrinogengehalt wird dabei die Gerinnungszeit verwendet. Aus diesem Grunde ist dieses Verfahren nicht automatisierbar. Zudem wird unter den genannten Reaktionsbedingungen die Bestimmung durch Fibrinogenspaltprodukte gestört.

Aus DD-A-158 954 ist ein Verfahren zur Bestimmung von Fibrinmonomeren mittels Latices aus Polystyrol nach der Agglutinationsmethode beschrieben, wobei gegebenenfalls ein Schlagengiftenzym zur Umwandlung des Fibrinogens verwendet wird. Dieses Verfahren ist jedoch sehr umständlich, da zunächst Fibrinogen in Fibrinmonomer überführt werden muß. Anschließend muß dann genau diejenige Probenverdünnung gefunden werden, bei der die Latices gerade nicht mehr agglutinieren. Dieser Verdünnungsgrad ist dann das Maß für die Fibrinogenmenge der Probe.

In US-A-4 245 039 ist ein Verfahren beschrieben, bei dem Fibrinogenspaltprodukte und/oder Fibrinspaltprodukte mit stabilisierten Gärmedien von Staphylococcus bestimmt werden können. Dabei wird die Menge der Spaltprodukte durch den Grad der Staphylococcen-Agglutination bestimmt. Dieses Verfahren ist nicht automatisierbar und außerdem zur Bestimmung von Fibrinogen ungeeignet.

Die folgenden Beispiele erläutern die Erfindung weiter.

**Beispiel 1**

Es werden die folgenden Lösungen hergestellt:

Lösung 1: 10 E Batroxobin werden in 1 ml Wasser gelöst.

Lösung 2: Es wird eine Lösung hergestellt, die 0,1 mol/l Tris/HCl pH 7,5, 5 mmol/l CaCl$_2$ sowie 1 % BriJ 35 und 2 % Polyethylenglykol 6000 enthält.

Lösung 3: (Reaktionsgemisch:) Es werden 90 µl von Lösung 1 mit 100 ml von Lösung 2 gemischt.

Eine photometrische Messung wird unter folgenden Bedingungen durchgeführt:

Photometer mit Schreiber, Wellenlänge 340 nm, Halbmikroküvette mit Schichtdicke 1 cm, Temperatur 25°C, Schreibergeschwindigkeit 1 cm/min.

Es werden 0,1 ml der fibrinogenhaltigen Plasmaprobe vorgelegt und die Reaktion durch Zugabe von 1,0 ml des auf 25°C erwärmten Reaktionsgemisches gestartet, wobei gleichzeitig der Schreiber gestartet wird. Aus dem linearen Teil des sich ergebenden Schreiberdiagramms wird die Extinktionsänderung pro Zeiteinheit abgelesen. Das Verfahren wird mit einer Lösung von bekanntem Fibrinogengehalt wiederholt. Aus den abgelesenen Extinktionsänderungen läßt sich die Konzentration der unbekannten Probe errechnen.

## Beispiel 2

30 Citratplasmen wie sie im Routinebetrieb eines Krankenhauses anfallen, werden nach Beispiel 1 analysiert. Dieselben Plasmen werden auch nach einer üblichen gerinnungsphysiologischen Routinemethode (Clauss-Test) analysiert. Der Methodenvergleich ergibt einen Korrelationskoeffizienten von r = 0.95.

## Beispiel 3

Menschliches Citratplasma mit einem Fibrinogengehalt von ca. 400 mg/dl wird auf 37°C erwärmt und mit Streptokinase (100 E/ml) versetzt. Zu bestimmten Zeiten werden Aliquote mit Aprotinin (3000 KI/ml) versetzt und die Wiederfindung an Fibrinogen mit zwei bekannten Methoden sowie mit der erfindungsgemäßen Methode bestimmt. Das Ergebnis zeigt Tabelle 1. Die in den Proben tatsächlich vorhandene Fibrinogenmenge ist durch die Methode nach Ratnoff-Menzie (Spalte 2) gegeben, welche allgemein als störunanfällige Referenzmethode angesehen wird. Die beiden anderen Methoden zeigen mit zunehmendem Anteil an Spaltprodukten reduzierte Wiederfindung; die erfindungsgemäße jedoch eine deutlich bessere als die Routinemethode nach Clauss.

## Tabelle 1

Wiederfindung von Fibrinogen in Plasmen, die mit Streptokinase behandelt wurden. Referenzmethode nach Ratnoff-Menzie. Mittel aus drei Experimenten.

| Abbauzeit (min) | Referenz- methode % | Kinet. Trübungs- test gem. Erfindung % | Clauss Test % |
|---|---|---|---|
| 0 | 100 | 100 | 100 |
| 2 | 95,7 | 95,1 | 94,9 |
| 5 | 91,0 | 88,0 | 78,9 |
| 10 | 83,5 | 67,4 | 53,3 |
| 20 | 61,5 | 41,5 | 30,2 |
| 40 | 46,5 | 28,8 | 19,0 |
| 60 | 41,2 | 24,3 | 12,8 |

## Beispiel 4

Citratplasma wird einem fibrinolytischen Abbau entsprechend Beispiel 3 unterworfen, wobei Aliquote wiederum zu entsprechenden Zeiten entnommen und mit Aprotinin gestoppt werden. Der Spaltproduktgehalt der Proben ist durch die Differenz aus dem ursprünglichen Fibrinogengehalt und dem Fibrinogengehalt der Proben (bestimmt nach Ratnoff-Menzie) gegeben. An den Proben wird weiterhin mittels des erfindungsgemäßen Verfahrens die Zeit bestimmt, die nach dem Start der Reaktion bis zum Einsetzen einer Trübungsänderung verstreicht. Wenn der Spaltproduktgehalt der Proben gegen diesen Meßwert aufgetragen wird, so erhält man eine lineare Beziehung (Tabelle 2) mit einem Korrelationskoeffizienten von r = 0,99; d. h. der untersuchte Parameter ist ein Maß für die Konzentration der in der Probe vorliegenden Spaltprodukte.

**Tabelle 2**

Beziehung zwischen FSP-Konzentration und Lag-Phase im kinetischen Trübungstest gemäß Erfindung.

| Abbauzeit (min) | FSP mg/dl | Reaktionszeit (lag-Phase) (sec) |
|---|---|---|
| 0 | 0 | 7,2 |
| 2 | 20,0 | 13,5 |
| 5 | 19,0 | 26,4 |
| 10 | 25,6 | 36,9 |
| 15 | 38,0 | 85,2 |
| 20 | 61,3 | 125 |
| 40 | 114 | 242 |
| 60 | 140 | 318 |
| 120 | 192 | 425 |

**Patentansprüche**

1. Verfahren zur gleichzeitigen Bestimmung von Fibrinogen und Fibrinogen-Spaltprodukten im Plasma, dadurch gekennzeichnet, daß man 0,01 bis 1 U eines Schlangengift-Enzyms mit thrombinartiger Wirksamkeit je ml Probeplasma zusetzt, dann die Zeitdauer zwischen dem Enzymzusatz und dem Beginn einer Trübungsbildung als Maß für die Menge an Fibrinogenspaltprodukten bestimmt und anschließend die Geschwindigkeit der Trübungsbildung als Maß für die Fibrinogenmenge mißt.

2. Verfahren nach Anspruch 1, dadurch gekennzeichnet, daß man Batroxobin, das Giftenzym der malaiischen Grubenotter (Agkistrodon Rhodostoma) oder das Giftenzym der Kupferkopfschlange (Agkistrodon Contortrix) verwendet.

3. Verfahren nach einem der vorhergehenden Ansprüche, dadurch gekennzeichnet, daß man 0,5 bis 4 Gew.-% eines wasserlöslichen Polyalkohols zusetzt.

4. Verfahren nach einem der vorhergehenden Ansprüche, dadurch gekennzeichnet, daß man die Bestimmung bei einem pH-Wert zwischen 7,0 und 9,0 durchführt.

5. Verfahren nach Anspruch 3 oder 4, dadurch gekennzeichnet, daß man als Polyalkohol Stärke, Dextran, Polyvinylalkohol, Polyäthylenglycol oder Zucker oder ein Zuckerpolymerisat verwendet.

6. Verfahren nach Anspruch 5, dadurch gekennzeichnet, daß man ein Polyethylenglycol mit einem Molekulargewicht zwischen 4000 und 22000 zusetzt.

7. Verfahren nach einem der vorhergehenden Ansprüche, dadurch gekennzeichnet, daß man in 0,05 bis 0,3 mol/l Pufferlösung arbeitet.

8. Verfahren nach einem der vorhergehenden Ansprüche, dadurch gekennzeichnet, daß man $Ca^{++}$-Ionen in einer Menge von 1 bis 20 mmol/l zusetzt.

9. Reagenz zur Durchführung des Verfahrens nach Anspruch 1 bis 8, dadurch gekennzeichnet, daß es 0,01 bis 11 U Schlangengiftenzym mit thrombinartiger Wirksamkeit je ml Probeplasma, 0,5 bis 4 Gew.-% eines wasserlöslichen Polyalkohols, 1 bis 20 mmol/l $Ca^{++}$ und 0,05 bis 0,3 mol/l Puffer pH 7,0 bis 8,0, bezogen auf die gebrauchsfertige Lösung und gegebenenfalls ein Detergenz, enthält.

**0 137 269**

**Revendications**

1. Procédé de détermination simultanée du fibrinogène et des produits de clivage du fibrinogène dans le plasma, caractérisé en ce qu'on ajoute, par ml d'échantillon de plasma, 0,01 à 1 unité d'une enzyme de venin de serpent ayant une activité analogue à celle de la thrombine, puis en ce qu'on détermine le laps de temps s'écoulant entre l'addition de l'enzyme et le début d'une formation de trouble en tant que mesure de la quantité de produits de clivage du fibrinogène et en ce que l'on mesure ensuite la vitesse de formation du trouble en tant que mesure de la quantité de fibrinogène.

2. Procédé selon la revendication 1, caractérisé en ce que l'on utilise la batroxobine, l'enzyme du venin du crotale malais (Agkistrodon Rhodostoma), ou l'enzyme du venin du "serpent à tête de cuivre" (Agkistrodon Contortrix).

3. Procédé suivant l'une des revendications précédentes, caractérisé en ce que l'on ajoute 0,5 à 4 % en poids d'un polyalcool soluble dans l'eau.

4. Procédé suivant l'une des revendicatons précédentes, caractérisé en ce que l'on effectue le dosage à une valeur de pH entre 7,0 et 9,0.

5. Procédé suivant la revendication 3 ou 4, caractérisé en ce que l'on utilise comme polyalcool l'amidon, le dextrane, l'alcool polyvinylique, le polyéthylène-glycol ou le sucre ou un polymère de sucre.

6. Procédé suivant la revendication 5, caractérise en ce que l'on ajoute un polyéthylène-glycol ayant un poids moléculaire entre 4000 et 22000.

7. Procédé suivant l'une des revendications précédentes, caractérisé en ce que l'on travaille dans une solution tampon à 0,05 à 0,3 mole/l.

8. Procédé suivant l'une des revendications précédentes, caractérisé en ce que l'on ajoute des ions Ca++ en une quantité de 1 à 20 mmoles/l.

9. Réactif pour l'exécution du procédé selon les revendications 1 à 8, caractérisé en ce qu'il contient 0,01 à 1 unité d'enzyme de venin de serpent ayant une activité analogue à celle de la thrombine par ml d'échantillon de plasma, 0,5 à 4 % en poids d'un polyalcool soluble dans l'eau, 1 à 20 mmoles/l de Ca++ et 0,05 à 0,3 mole/l de tampon pH 7,0 à 8,0, par rapport à la solution prête à l'emploi, et le cas échéant un détergent.

**Claims**

1. Process for the simultaneous determination of fibrinogen and fibrinogen fission products in plasma, characterised in that one adds 0.01 to 1U of a snake venom enzyme with a thrombin-like effectiveness per ml. of sample plasma, then determines the period of time between the enzyme addition and the commencement of a turbidity formation as a measure for the amount of fibrinogen fission products and subsequently measures the speed of the turbidity formation as a measure of the amount of fibrinogen.

2. Process according to claim 1, characterised in that one uses batroxobin, the venom enzyme of the Malayan pit viper (Agkistrodon rhodostoma) or the venom enzyme of the copperhead snake (Agkistrodon contortix).

3. Process according to one of the preceding claims, characterised in that one adds 0.5 to 4 wt.% of a watersoluble polyalcohol.

4. Process according to one of the preceding claims, characterised in that one carries out the determination at a pH value between 7.0 and 9.0.

5. Process according to claim 3 or 4, characterised in that, as polyalcohol, one uses starch, dextran, polyvinyl alcohol, polyethylene glycol or sugar or a sugar polymer.

6. Process according to claim 5, characterised in that one adds a polyethylene glycol with a molecular weight between 4000 and 22,000.

7. Process according to one of the preceding claims, characterised in that one works in 0.05 to 0.3 mole/l. buffer solution.

8. Process according to one of the preceding claims, characterised in that one adds Ca++ ions in an amount of 1 to 20 mMole/l.

9. Reagent for carrying out the process according to claims 1 to 8, characterised in that it contains 0.01 to 1 U snake venom enzyme with thrombin-like effectiveness per ml. of sample plasma, 0.5 to 4 wt.% of a watersoluble polyalcohol, 1 to 20 mMole/l. Ca++ ions and 0.05 to 0.3 mole/l. of buffer pH 7.0 to 8.0, referred to the solution ready for use, and optionally a detergent.